# EUROPÄISCHE PATENTSCHRIFT

(11) **EP 1 029 866 B1**
(45) Veröffentlichungstag und Bekanntmachung des Hinweises auf die Patenterteilung: **04.12.2002**
(21) Anmeldenummer: 00102141.9
(22) Anmeldetag: 07.02.2000
(51) Int. Cl.: C07F 9/165, C08K 5/5398

(54) **Verfahren zur Herstellung von Dithiophosphorsäurepolysulfid-Gemischen**
Process for the preparation of dithiophosphoricpolysulfide mixtures
Procédé pour la préparation de mélanges de polysulfures dithiophosphoriques

(30) Priorität: 19.02.1999 DE 19906986
(43) Veröffentlichungstag der Anmeldung: 23.08.2000
(73) Patentinhaber: Rhein Chemie Rheinau GmbH, 68219 Mannheim (DE)
(72) Erfinder: Heiliger, Ludger, Dr., 67433 Neustadt (DE); Pauli, Alfred, Dr., 68799 Reilingen (DE); Hegmann, Joachim, Dr., 67117 Limburgerhof (DE); Schudok, Clemens, Dr., 67240 Bobenheim-Roxheim (DE); Früh, Thomas, Dr., 67061 Ludwigshafen (DE)
(74) Vertreter: Bailly, Peter

(56) Entgegenhaltungen:
- EP-A- 0 383 102
- DD-A- 228 722
- DE-A- 2 249 090
- US-A- 2 237 627

## Beschreibung

Die Erfindung bezieht sich auf ein neues Verfahren zur Herstellung von Dithiophosphorsäurepolysulfid-Gemischen ausgehend von Dithiophosphorsäuredisulfid.

Dithiophosphorsäurepolysulfide sind bekannt, ebenso ihre Verwendung als Vulkanisationsmittel oder Vulkanisationsbeschleuniger für die Kautschukvulkanisation (vgl. DE 19 36 694, DE 22 49 090 sowie DE 44 31 727). Dithiophosphorsäurepolysulfide können hergestellt werden aus den entsprechenden Dithiophosphorsäuren bzw. deren Alkalisalzen und Schwefelchloriden, wie Dischwefeldichlorid oder Schwefeldichlorid.

Nachteilig bei der Umsetzung der Dithiophosphorsäuren mit Schwefelchloriden ist die hohe Korrosivität der Schwefelchloride und deren unangenehmer Geruch, was zu Handling-Problemen führt und entsprechende, aufwendige Apparaturen erforderlich macht. Gemäß EP 0 383 102 A1 disproportioniert Schwefeldichlorid außerdem sehr leicht, so daß nach diesem Verfahren die Dithiophosphorsäuretrisulfide direkt nicht zugänglich sind.

Zusätzlich neigen die nach dem zuvor beschriebenen Verfahren erhaltenen Dithiophosphorsäuretetrasulfide aufgrund ihrer geringeren Stabilität zur Schwefelabspaltung. Um solches zu verhindern ist es erforderlich, die Dithiophosphorsäuretetrachloride gegen eine Schwefelausfällung zu stabilisieren, wie es beispielsweise in DE 44 31 727 beschrieben wird.

Aufgabe der vorliegenden Erfindung ist es nun, ein Verfahren zur Herstellung von Dithiophosphorsäurepolysulfid-Gemischen zur Verfügung zu stellen, das den Einsatz von Schwefelchloriden vermeidet und zu schwefelstabilen Dithiophosphorsäurepolysulfidgemischen führt.

Gegenstand der Erfindung ist daher ein Verfahren zur Herstellung von Dithiophosphorsäurepolysulfid-Gemischen der Formel 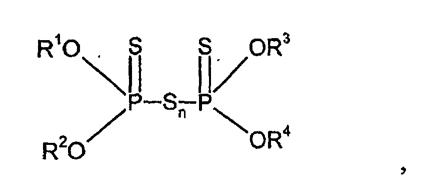 worin
- R¹ bis R⁴: gleich oder verschieden sind und für einen geradkettigen oder verzweigten C₁-C₁₈-Alkylrest, C₁-C₁₈-Alkenylrest, C₅-C₂₈-Cycloalkylrest, C₅- C₂₈-Cycloalkenylrest sowie einen C₆-C₂₈-Arylrest oder C₇-C₂₈-Aralkylrest stehen
und
- n: für Zahlen von 2,5 bis bis 3,5 steht,
das dadurch gekennzeichnet ist, daß man Dithiophosphorsäuredisulfide der Formel 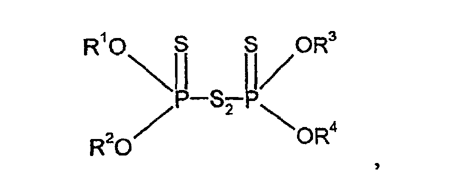 worin
- R¹ bis R⁴: die obengenannte Bedeutung besitzen,
mit 0,5 bis 1,5 Mol Schwefel, gegebenenfalls in Gegenwart eines Lösungsmittels, bei Temperaturen von 100 bis 140°C umsetzt.

Die Zahlenwerte für n ergeben sich durch statistische Ermittlung, entsprechend der Schwefel-Kettenverteilung im Molekül.

Die Reste R¹ bis R⁴ der obengenannten Formeln können durch geeignete, für die spätere Verwendung der Polysulfide nicht störende Reste substituiert sein, wobei insbesondere Alkylreste und Cycloalkylreste zu nennen sind.

Bevorzugt sind als Reste R¹ bis R⁴ C₆-C₁₂-Alkylreste, insbesondere C₈-C₁₂-Alkylreste, zu nennen, insbesondere verzweigte Alkylreste, wie 2-Ethyl-hexyl.

Bevorzugt werden nach dem erfindungsgemäßen Verfahren Dithiophosphorsäurepolysulfidgemische hergestellt, in denen n für Zahlen von 2,8 bis 3,3 steht.

Die in das erfindungsgemäße Verfahren einzusetzenden Dithiophosphorsäuredisulfide sind ebenfalls bekannt und beispielsweise beschrieben im Journal of Applied Polymer Science, Vol. 19, pp. 865-877 (1975). Hergestellt werden die Disulfide beispielsweise durch Oxidation von Dithiophosphorsäure mit Wasserstoffperoxid oder HOCl oder einer Mischung aus Kaliumbromid und Wasserstoffperoxid.

Bei dem erfindungsgemäßen Verfahren werden die eingesetzten Disulfide bevorzugt mit 0,8 bis 1,3 Mol elementarem Schwefel umgesetzt, wobei Temperaturen von 110 bis 130°C, insbesondere 120 bis 130°C, bevorzugt sind.

Falls die Umsetzung in Lösung durchgeführt werden soll, werden als Lösungsmittel insbesondere aliphatische Lösungsmittel, beispielsweise Benzin, aromatische Lösungsmittel, z.B. Toluol, oder halogenierte aromatische Lösungsmittel, wie Chlorbenzol, eingesetzt. Die Lösungsmittel können selbstverständlich auch im Gemisch untereinander eingesetzt werden.

Die Menge an Lösungsmittel kann leicht durch entsprechende Vorversuche bestimmt werden. Üblicherweise sind Mengen an Lösungsmittel von 0,1 bis 50 Gew.-%, bezogen auf die Gewichtsmenge des eingesetzten Disulfids.

Weiterhin ist es möglich, nach erfindungsgemäßer Umsetzung Wasserstoffperoxid zuzusetzen, um gegebenenfalls auftretende Farbveränderungen zu verhindern. Dabei wird das Wasserstoffperoxid in Mengen von 0,1 % bis 5 Gew.-%, bezogen auf die Gewichtsmenge des eingesetzten Disulfids eingesetzt.

Abhängig von den angewandten Reaktionsbedingungen beträgt die Reaktionszeit ca. 10 Minuten bis ca. 6 Stunden.

Da die erfindungsgemäß hergestellten Dithiophosphorsäurepolysulfide besonders schwefelstabil sind, d.h. nicht zur Absetzung von Schwefelkristallen neigen, eignen sie sich besonders für den Einsatz als Schwefelspender für die Vulkanisation von Natur- und Synthesekautschuk sowie für die Latex-Vulkanisation von Natur- und Synthesekautschuk-Latex.

### Beispiele

### Beispiel 1

999,0 g (2,6179 Mol) O,O-Di-2-ethylhexyldithiophosphorsäurediester (C₈-DTPS) mit einer Säurezahl von 147 mg KOH/g und 1,0 g (0,0084 Mol) Kaliumbromid werden in einem mit Rührer, Tropftrichter, Gaseinleitung und Rückflußkühler versehenen Kolben bei Raumtemperatur vorgelegt und auf 30°C temperiert.

Anschließend werden 163,2 g (1,4399 Mol) Wasserstoffperoxid so zugetropft, daß die Temperatur bei dieser stark exothermen Reaktion nicht über 40°C ± 5°C ansteigt.

Zur Beschleunigung der Phasentrennung werden 50 g Benzin (80-110) und 10 g Natriumchlorid zugegeben und der Ansatz bei 40°C ± 5°C stehen gelassen, bis die Phasen sauber getrennt sind.

Die unten befindliche wässrige Phase wird abgetrennt. Die gelbe, trübe organische Phase wird auf 70°C ± 5°C erwärmt und im Vakuum (20 bis 60 mbar) destilliert. Anschließend wird über 1 % Kieselgur filtriert.

Für die zweite Reaktionsstufe wird der Ansatz 5 Minuten mit Stickstoff ausgeblasen. Danach werden 46,1 g (1,4399 mol) Schwefel zugegeben und die Reaktionsmischung auf 122°C ± 2°C erwärmt, um den Schwefel einzukochen. Der Ansatz wird vier Stunden lang bei dieser Temperatur gerührt.

Anschließend kann das gelbe Produkt ohne Filtration abgefüllt werden. Das Produkt zeigt auch nach mehreren Monaten keine Trübung oder Ausscheidungen.

Die Kettenverteilung in dem erhaltenen Dithiophosphorsäurepolysulfid-Gemisch wurde durch Hochdruck-Flüssigkeits-Chromatographie (HPLC) bestimmt und setzt sich wie folgt zusammen:

| | | | | |
|---|---|---|---|---|
| n=1 | 0,8 % | n=4 | 19,9 % | n = 7 2,5 %, jeweils bezogen auf die Flächen der HPLC-Signale |
| n=2 | 29,2% | n=5 | 8,1% | |
| n=3 | 35,5% | n=6 | 4,4% | |

### Vergleichsbeispiel 1

6 000 g (15 Mol) O,O-Di-2-ethylhexyldithiophosphorsäurediester (C₈-DTPS) mit einer Säurezahl von 139,8 mg KOH/g und 300 g Benzin 80-100 werden bei Raumtemperatur vorgelegt. Innerhalb von 1,5 h werden 2553,2 g 23,5 %ige (15,0 mol) Natronlauge zugegeben, wobei die Temperatur auf 45°C ansteigt. Anschließend wird auf 55°C hochgeheizt, um dann innerhalb von 5 h 1 003 g (7,4 mol) S₂-Cl₂ zuzutropfen. Zur Komplettierung der Reaktion wird noch 30 Minuten nachgerührt.

Anschließend wird der Ansatz mit ca. 600 g 8 %ige NaHCO₃-Lösung auf pH 7 eingestellt. Um eine gute Phasentrennung zu erreichen, wird auf 65°C aufgeheizt und 1 200 g Wasser zugegeben.

Nach der Abtrennung der wässrigen Phase wird das Produkt 3 h bei 70°C im Wasserstrahlvakuum andestilliert. Anschließend wird unter Verwendung eines Filterhilfsstoffes über eine beheizte Druckfilternutsche filtriert. Das Produkt zeigt bereits nach wenigen Stunden gelbe kristalline Ausscheidungen, die sich als Elementarschwefel herausstellen.

### Vergleichsbeispiel 2

6 000 g (15 Mol) O,O-Di-2-ethylhexyldithiophosphorsäurediester (C₈-DTPS) mit einer Säurezahl von 139,8 mg KOH/g und 300 g Benzin 80-100 werden bei Raumtemperatur vorgelegt. Innerhalb von 1,5 h werden 2553,2 g 23,5 %ige (15,0 mol) Natronlauge zugegeben, wobei die Temperatur auf 45°C ansteigt. Anschließend wird auf 55°C hochgeheizt, um dann innerhalb von 5 h 1 003 g (7,4 mol) S₂-Cl₂ zuzutropfen. Zur Komplettierung der Reaktion wird noch 30 Minuten nachgerührt.

Anschließend wird der Ansatz mit ca. 600 g 8 %ige NaHCO₃-Lösung auf pH 7 eingestellt. Um eine gute Phasentrennung zu erreichen, wird auf 65°C aufgeheizt und 1 200 g Wasser zugegeben.

Nach der Abtrennung der wässrigen Phase werden bei 65°C 282 g 2-Ethylhexansäure und 180 g Zinkoxid zur Stabilisierung gegen Schwefelausfällung portionsweise zudosiert. Nach beendeter Zugabe wird noch 2 h bei 70°C nachreagiert.

Zur Trocknung wird das Produkt 3 h bei 70°C im Wasserstrahlvakuum andestilliert. Abschließend wird unter Verwendung eines Filterhilfsstoffes über eine beheizte Druckfilternutsche filtriert. Das Produkt zeigt bereits nach einigen Tagen gelbe kristalline Ausscheidungen, die sich als Elementarschwefel herausstellen.

## Patentansprüche

1. Verfahren zur Herstellung von Dithiophosphorsäurepolysulfid-Gemischen der Formel 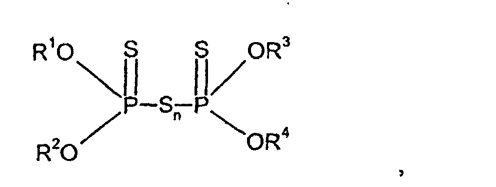 worin
R¹ bis R⁴ gleich oder verschieden sind und für einen geradkettigen oder verzweigten C₁-C₁₈-Alkylrest, C₁-C₁₈-Alkenylrest, C₅-C₂₈-Cycloalkylrest, C₅- C₂₈-Cycloalkenylrest sowie einen C₆-C₂₈-Arylrest oder C₇-C₂₈-Aralkylrest stehen
und
n für Zahlen von 2,5 bis bis 3,5 steht,
das **dadurch gekennzeichnet ist, daß** man Dithiophosphorsäuredisulfide der Formel 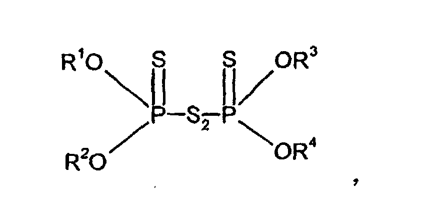 worin
R¹ bis R⁴ die obengenannte Bedeutung besitzen,
mit 0,5 bis 1,5 Mol Schwefel, gegebenenfalls in Gegenwart eines Lösungsmittels, bei Temperaturen von 100 bis 140°C umsetzt.

## Claims

1. Process for the production of dithiophosphoric acid polysulfide mixtures of the formula 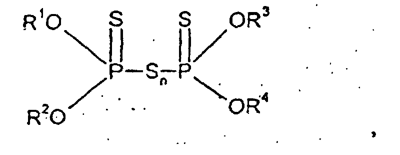 in which
R¹ to R⁴ are the same or different and represent a straight-chain or branched C₁ - C₁₈ alkyl group, C₁ - C₁₈ alkenyle group, C₅ - C₂₈ cycloalkyl group, C₅ - C₂₈ cycloalkenyle group and also a C₆ - C₂₈ aryl group or C₇ - C₂₈ aralkyl group
and
n represents numbers from 2.5 to 3.5,
**characterised in that** dithiophosphoric acid disulfides of the formula 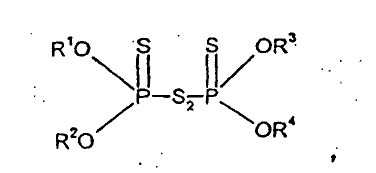 in which
R¹ to R⁴ have the meaning given above
are reacted with 0.5 to 1.5 mol sulfur, optionally in the presence of a solvent, at temperatures of 100 to 140°C.

## Revendications

1. Procédé pour la préparation de mélanges de polysulfures dithiophosphoriques de formule 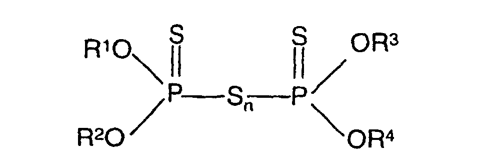 dans laquelle
R¹ à R⁴, ayant des significations identiques ou différentes, représentent chacun un groupe alkyle à chaîne droite ou ramifiée en C₁-C₁₈, un groupe alcényle en C₁-C₁₈, cycloalkyle en C₅-C₂₈, cycloacényle en C₅-C₂₈, ou un groupe aryle en C₆-C₂₈ ou aralkyle en C₇-C₂₈,
et
n est un nombre allant de 2,5 à 3,5,
ce procédé se caractérisant en ce que l'on fait réagir des disulfures dithiophosphoriques de formule 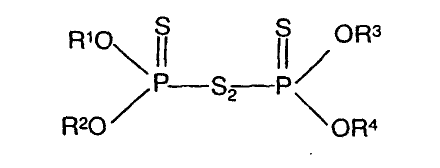 dans laquelle
R¹ à R⁴ ont les significations indiquées ci-dessus,
avec 0,5 à 1,5 mol de soufre, le cas échéant en présence d'un solvant, à des températures de 100 à 140°C.
